# EUROPEAN PATENT SPECIFICATION

(11) **EP 1 545 702 B1**
(45) Date of publication and mention of the grant of the patent: **13.02.2008**
(21) Application number: 03797781.6
(22) Date of filing: 18.09.2003
(51) Int. Cl.: A61N 1/378, A61N 1/375

(54) **HARMLESS WIRELESS ENERGY TRANSMISSION TO IMPLANT**
HARMLOSE DRAHTLOSE ENERGIEÜBERTRAGUNG AN EIN IMPLANTAT
TRANSMISSION SANS FIL D'ENERGIE INOFFENSIVE VERS UN IMPLANT

(30) Priority: 20.09.2002 US 412014 P
(43) Date of publication of application: 29.06.2005
(73) Proprietor: Potencia Medical AG, 6341 Baar (CH)
(72) Inventor: FORSELL, Peter, 6300 Zug (CH)
(74) Representative: Strandin, Heléne
(86) International application number: PCT/SE2003/001464
(87) International publication number: WO 2004/026399

(56) References cited:
- US-A- 4 071 032
- US-A- 4 723 536
- US-B1- 6 389 318

## Description

The present invention relates to an apparatus for wireless transfer of energy from outside a human's or animal's body to an energy consuming medical device implanted in the human's or animal's body. The apparatus comprises a transmission device operable from outside the human's or animal's body for transmission of an alternating magnetic field to a receiver implantable in the human's or animal's body for receiving and drawing energy from the alternating magnetic field to be supplied to the energy consuming implanted medical device.

Such a transmission device typically includes a coil that wirelessly co-operates with a coil of the implanted receiver. It has been found, however, that it is very unpleasant to a person to operate a handheld transmission device, because bad sensations occur in the person's hand while the transmission device is transmitting the alternating magnetic field. These bad sensations were not reduced significantly when the transmission device was encapsulated in a plastic case with a certain distance between the hand and the coil. Probably, the alternating magnetic field somehow unfavorably influences the nerves of the hand. It is not possible to shield the hand from the magnetic field by providing a steel shield between the hand and the coil, because such a steel shield would shorten the magnetic field. Because of the above-noted problems, prior hand-held transmission devices of coil-to-coil type have not been commercialized.

US-B1-6 389 318 discloses a magnetic shield in the form of a circular magnetizable flat casing adapted to shield the environment from an alternating magnetic field generated by a primary coil for transferring energy through a patient's skin to an implanted medical device. The flat casing surrounds the rear end of the primary coil.

The object of the present invention is to provide an apparatus for wireless transfer of energy including a magnetic field transmission device, which at least significantly reduces or even prevents unpleasant sensations to a person's hand during manual operation of the transmission device.

Another object of the invention is to provide an apparatus for wireless transfer of energy including a magnetic field transmission device, which at least significantly reduces or even prevents environmental disturbances during operation of the transmission device.

Yet another object of the invention is to propose a use of the new apparatus.

Accordingly, there is provided an apparatus for wireless transfer of energy from outside a human's or animal's body to an energy consuming medical device implanted in the human's or animal's body, comprising a transmission device operable from outside the human's or animal's body for transmission of an alternating magnetic field, a receiver implantable in the human's or animal's body for receiving and drawing energy from the alternating magnetic field to be supplied to the energy consuming implanted medical device, the transmission device including at least one coil for generating the alternating magnetic field in a desired direction towards the receiver, the coil having a longitudinal extension, a front end to be directed towards the receiver and a rear end to be directed away from the receiver, and at least one shield adapted to shield the environment from the alternating magnetic field generated by the coil, the shield including a magnetizable casing surrounding the rear end of the coil. The apparatus is characterized in that the shield includes a magnetizable core extending in the coil, and that the magnetizable casing is integrated with the core and surrounds the circumference of the coil along at least a portion of the longitudinal extension of the coil.

The shield reduces the peaks or transients of the alternating magnetic field and the magnetic field itself in the direction towards, for example, an operator's hand while the transmission efficiency of the transmission device in the opposite direction, i.e. the direction towards the implanted receiver, is maintained. As a result, the unpleasant hand sensations previously experienced by operators testing prior hand-held transmission devices are practically eliminated by the transmission device of the invention. This improvement of the transmission device of the invention is of great significance, because the transmission device is intended for use daily by an operator, such as a doctor or nurse, to treat different patients with energy consuming implants, and a doctor or nurse would be very reluctant to use a transmission device that gives rise to the unpleasant hand sensations discussed above.

The casing may completely surround the coil except the front end thereof and the core may wholly extend along the longitudinal extension of the coil.

Alternatively, the casing may surround the circumference of the coil along a portion of the longitudinal extension of the coil and the core or/and coil may extend past the casing along the longitudinal extension of the coil, as seen in the direction towards the front end of the coil.

In accordance with an embodiment of the present invention, the casing includes a circular cylindrical wall and a circular gable wall joined to the cylindrical wall. The core extends centrally in the cylindrical wall from the gable wall and the coil is applied on the core with the rear end of the coil facing the gable wall. The cylindrical wall may be provided with cutouts, in order not to reduce the effective magnetic field too much. Alternatively, the cylindrical wall may be shorter than the core. For example, the cylindrical wall may extend axially from the gable wall along half the length of the core or a third of the length of the core. Depending on the strength of the transmitted magnetic field the length of the cylindrical wall may be more reduced or even eliminated. The coil may be shorter or longer than the core and/or the cylindrical wall.

In all embodiments of the invention, the shield preferably is made of ferrite or similar magnetizable material.

The transmission device suitably includes a plastic box, in which the coil and shield are arranged such that they are located at a distance, in the order of centimeters, from the operator's hand, when the operator holds the transmission device.

Alternatively, the transmission device may include two identical transmitters that are to be placed at different sides of the implanted receiver, for example on the human's abdomen and back. The two transmitters may also be placed on a stand relative to the human's or animal's body so that the two transmitters are in the desired positions for transmitting the alternating magnetic fields towards the implanted receiver.

The apparatus may be used for supplying energy to implanted medical devices, such as adjustable restriction devices for treating obesity, heartburn and reflux disease, urinary and anal incontinence, infusion pumps, muscle stimulators, impotence or other wireless transfer of energy to implants.

In accordance with another aspect of the present invention, the apparatus described above is used for transmitting the alternating magnetic field to a receiver implanted in a human's or animal's body, wherein the implanted receiver is capable of drawing energy from the alternating magnetic field to be supplied to an energy consuming medical device implanted in the human's or animal's body.

The present invention is described in more detail in the following with reference to the accompanying drawings, in which
Figure 1 schematically illustrates an apparatus of the present invention,
Figure 2 is a front view of a coil and shield of a hand-held transmission device of the apparatus according to an embodiment of the invention,
Figure 3 is a cross-section along the line III-III in Figure 2,
Figure 4 is a perspective view of a coil and shield of a hand-held transmission device of the apparatus according to another embodiment of the invention,
Figure 5 is a cross-section through the shield shown in Figure 4,
Figure 6 illustrates an embodiment of the apparatus of the invention used for transferring energy to an artificial sphincter applied on the rectum of an anal incontinent human being, and
Figure 7 is a modification of the embodiment according to Figure 6.

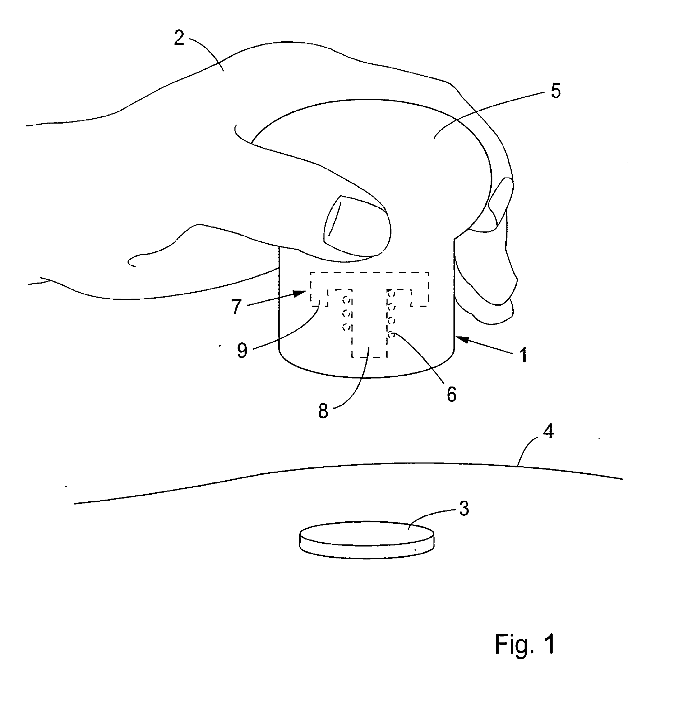

Figure 1 illustrates manual operation of an apparatus of the invention including an alternating magnetic field transmission device 1 held by an operator's hand 2 and a receiver 3 subcutaneously implanted in a human's body 4. The implanted receiver 3 is capable of receiving the alternating magnetic field transmitted by the transmission device 1 and of drawing energy from the alternating magnetic field to be supplied to an energy consuming medical device implanted in the body 4. The transmission device 1 includes a plastic box 5 containing a coil 6 adapted to generate an alternating magnetic field in the direction away from the hand 2 towards the implanted receiver 3. The coil 6 is situated in the lower part of the box 5 a few centimeters from the hand 2.

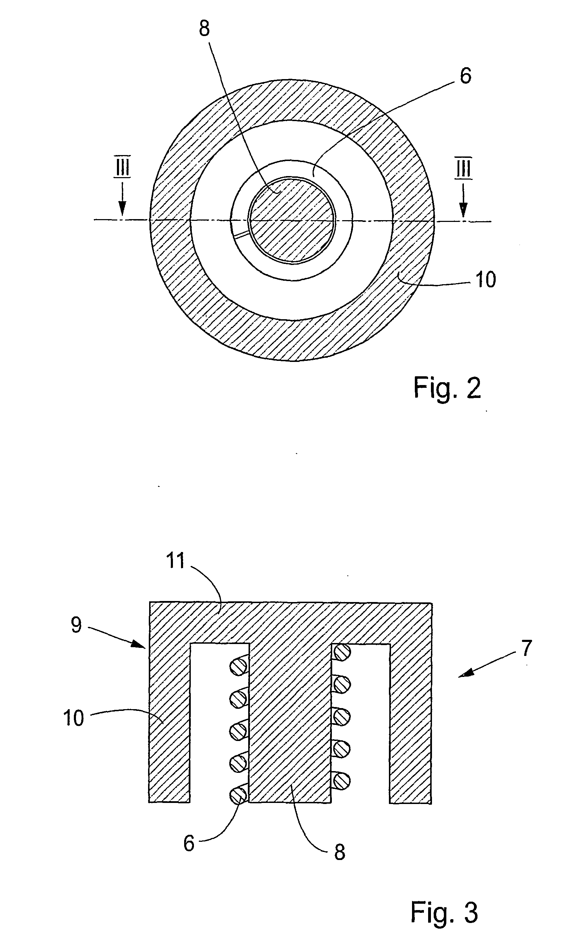

Figures 2 and 3 show a shield 7 of the apparatus for shielding the hand 2 from the alternating magnetic field including a ferrite core 8 extending in the coil 6 along the entire longitudinal extension thereof. (Alternatively, the coil 6 may be shorter than the core 8.) A ferrite casing 9 is integrated with the core 8 and surrounds the top rear end of the coil 6 and the circumference of the coil 6 along the entire longitudinal extension of the coil 6. The casing 9 includes a circular cylindrical wall 10 surrounding the coil 6 and a circular gable wall 11 joined to the cylindrical wall 10.

In Figure 1 the shield 7 has a somewhat modified design, the position of the shield 7 being indicated in dotted lines. Thus, the cylindrical wall 10 extends only halfway along the coil 6.

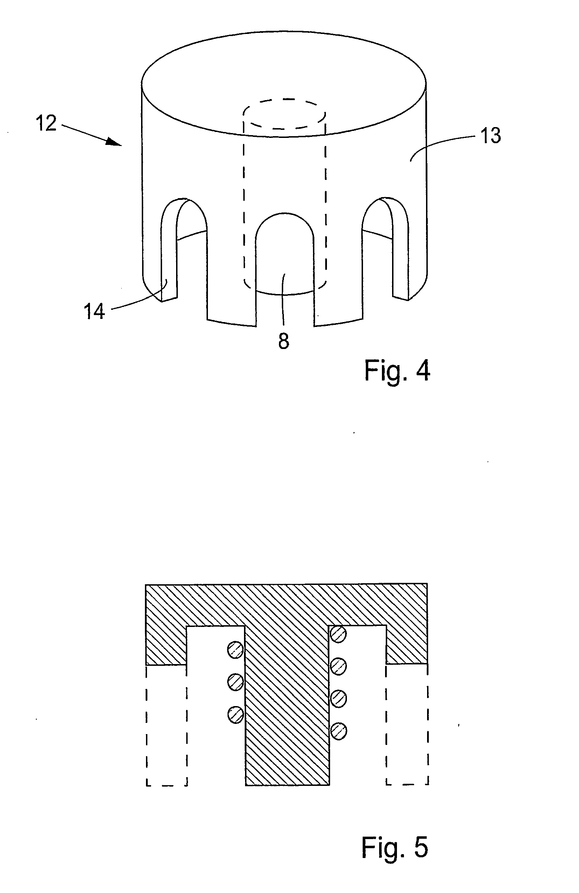

Figures 4 and 5 show a modified shield 12 that does not dampen the alternating magnetic field as much as the shield 7 of Figures 2 and 3. Thus, the cylindrical wall 13 of the shield 12 is provided with several cutouts 14 evenly distributed around the circumference of the cylindrical wall 13. In this embodiment, the coil 6 is shorter than the core 8.

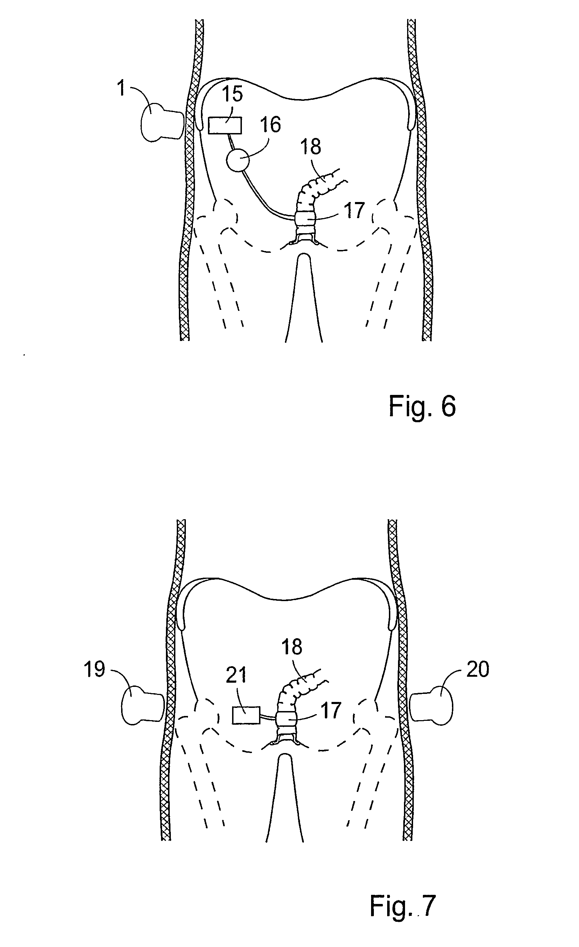

Figure 6 shows an embodiment of the apparatus used for an anal incontinent human. The apparatus includes the transmission device 1 and a receiver 15 subcutaneously implanted in the human's body. The receiver 15 supplies energy to an implanted operation device 16 that operates an artificial sphincter 17 applied on the human's rectum 18. The transmission device 1 is held by an operator who puts it on the human's skin substantially in front of the implanted receiver 15 to provide efficient energy transmission from the transmission device 1 to the receiver 15.

The embodiment shown in Figure 7 is identical to the embodiment shown in Figure 6, except that the transmission device 1 include two wireless energy transmitters 19 and 20, and the receiver and operation device are integrated in a single receiver/operation unit 21 located close to the artificial sphincter 17. Each transmitter 19,20 may be designed as the transmission device 1 described above in connection with Figs. 1-5. In this embodiment the transmitters 19 and 20 are positioned at different sides of the implanted receiver/operation unit 21, suitably at opposite sides as illustrated in Figure 7, to transmit wireless energy to the receiver/operation unit 21.

Of course, two wireless energy transmitters may also be used for transmitting energy to the receiver 15 of the embodiment shown in Figure 6.

## Claims

1. An apparatus for wireless transfer of energy from outside a human's or animal's body to an energy consuming medical device (17) implanted in the human's or animal's body, comprising a transmission device (1;19,20) operable from outside the human's or animal's body for transmission of an alternating magnetic field, a receiver (3;15;21) implantable in the human's or animal's body for receiving and drawing energy from the alternating magnetic field to be supplied to the energy consuming implanted medical device (17), the transmission device including at least one coil (6) for generating the alternating magnetic field in a desired direction towards the receiver, the coil having a longitudinal extension, a front end to be directed towards the receiver and a rear end to be directed away from the receiver, and at least one shield (7;12) adapted to shield the environment from the alternating magnetic field generated by the coil (6), the shield including a magnetizable casing (9) surrounding the rear end of the coil, **characterized in that** the shield includes a magnetizable core (8) extending in the coil (6), and that the magnetizable casing (9) is integrated with the core and surrounds the circumference of the coil along at least a portion of the longitudinal extension of the coil.

2. An apparatus according to claim 1, wherein the transmission device comprises a first transmitter (19) and a second transmitter (20), the first and second transmitters include first and second coils (6), respectively, for generating respective alternating magnetic fields in two different directions towards the receiver (21), each of the first and second coils having a longitudinal extension, a front end to be directed towards the receiver and a rear end to be directed away from the receiver, and the first and second transmitters include first and second shields (9) adapted to shield the environment from the alternating magnetic fields, each of the first and second shields including a magnetizable core (8) extending in the coil and a magnetizable casing (9) integrated with the core and surrounding the rear end of the coil and the circumference of the coil along at least a portion of the longitudinal extension of the coil.

3. An apparatus according to claim 1, wherein the casing (9) completely surrounds the coil (6) except the front end thereof.

4. An apparatus according to claim 3, wherein the core (8) wholly extends along the longitudinal extension of the coil (6).

5. An apparatus according to claim 1, wherein the casing (9) surrounds the circumference of the coil (6) along a portion of the longitudinal extension of the coil.

6. An apparatus according to claim 5, wherein the core (8) and/or coil (6) extends past the casing (9) along the longitudinal extension of the coil, as seen in the direction towards the front end of the coil.

7. An apparatus according to any one of claims 1, 3-6, wherein the casing (9) comprises a circular cylindrical wall (10;13) and a circular gable wall (11) joined to the cylindrical wall, the core (8) extends centrally in the cylindrical wall from the gable wall, and the coil is applied on the core with the rear end of the coil facing the gable wall.

8. An apparatus according to claim 7, wherein the cylindrical wall (13) is provided with cut-outs (14).

9. An apparatus according to any one of claims 1, 3-8, wherein the shield (7) is made of ferrite.

10. An apparatus according to any one of claims 1, 3-9, further comprising a plastic box (5), in which the coil (6) and shield (7) are arranged such that they are located at a distance, in the order of centimeters, from an operator's hand, when the operator holds the transmission device (1;19,20) during operation.

11. Use of the apparatus according to any one of claims 1-10 for transmitting the alternating magnetic field to a receiver implanted in a human's or animal's body, the implanted receiver (3;15;21) being capable of drawing energy from the alternating magnetic field to be supplied to an energy consuming medical device implanted in the human's or animal's body.

## Patentansprüche

1. Vorrichtung zur drahtlosen Übertragung von Energie von außerhalb eines Körpers eines Menschen oder eines Tiers zu einer Energie verbrauchenden medizinischen Vorrichtung (17), die in den Körper des Menschen oder des Tiers implantiert ist, mit einer Übertragungsvorrichtung (1; 19, 20), die von außerhalb des Körpers des Menschen oder des Tiers zur Übertragung eines Wechselmagnetfeldes betreibbar ist, einem Empfänger (3; 15; 21), der in den Körper des Menschen oder des Tiers implantierbar ist, zum Empfangen und Entnehmen von Energie aus dem Wechselmagnetfeld, die zur Energie verbrauchenden implantierten medizinischen Vorrichtung (17) geliefert werden soll, wobei die Übertragungsvorrichtung mindestens eine Spule (6) zum Erzeugen des Wechselmagnetfeldes in einer gewünschten Richtung zum Empfänger hin, wobei die Spule eine Längsausdehnung, ein in Richtung des Empfängers zu richtendes Vorderende und ein vom Empfänger weg zu richtendes Hinterende aufweist, und mindestens eine Abschirmung (7; 12) umfasst, die dazu ausgelegt ist, die Umgebung vor dem von der Spule (6) erzeugten Wechselmagnetfeld abzuschirmen, wobei die Abschirmung ein magnetisierbares Gehäuse (9) umfasst, das das Hinterende der Spule umgibt, **dadurch gekennzeichnet, dass** die Abschirmung einen magnetisierbaren Kern (8) umfasst, der sich in die Spule (6) erstreckt, und dass das magnetisierbare Gehäuse (9) mit dem Kern integriert ist und den Umfang der Spule entlang mindestens eines Teils der Längsausdehnung der Spule umgibt.

2. Vorrichtung nach Anspruch 1, wobei die Übertragungsvorrichtung einen ersten Sender (19) und einen zweiten Sender (20) umfasst, wobei der erste und der zweite Sender eine erste bzw. eine zweite Spule (6) zum Erzeugen von jeweiligen Wechselmagnetfeldern in zwei verschiedenen Richtungen zum Empfänger (21) hin umfassen, wobei jede der ersten und der zweiten Spule eine Längsausdehnung, ein in Richtung des Empfängers zu richtendes Vorderende und ein vom Empfänger weg zu richtendes Hinterende aufweist, und der erste und der zweite Sender eine erste und eine zweite Abschirmung (9) umfassen, die dazu ausgelegt sind, die Umgebung vor den Wechselmagnetfeldern abzuschirmen, wobei jede der ersten und der zweiten Abschirmung einen magnetisierbaren Kern (8), der sich in die Spule erstreckt, und ein magnetisierbares Gehäuse (9) umfasst, das mit dem Kern integriert ist und das Hinterende der Spule und den Umfang der Spule entlang zumindest eines Teils der Längsausdehnung der Spule umgibt.

3. Vorrichtung nach Anspruch 1, wobei das Gehäuse (9) die Spule (6) abgesehen von deren Vorderende vollständig umgibt.

4. Vorrichtung nach Anspruch 3, wobei sich der Kern (8) vollständig entlang der Längsausdehnung der Spule (6) erstreckt.

5. Vorrichtung nach Anspruch 1, wobei das Gehäuse (9) den Umfang der Spule (6) entlang eines Teils der Längsausdehnung der Spule umgibt.

6. Vorrichtung nach Anspruch 5, wobei sich der Kern (8) und/oder die Spule (6) am Gehäuse (9) vorbei entlang der Längsausdehnung der Spule in der Richtung zum Vorderende der Spule hin gesehen erstreckt.

7. Vorrichtung nach einem der Ansprüche 1, 3-6, wobei das Gehäuse (9) eine kreisförmige, zylindrische Wand (10; 13) und eine kreisförmige Giebelwand (11), die mit der zylindrischen Wand verbunden ist, umfasst, der Kern (8) sich zentral in der zylindrischen Wand von der Giebelwand erstreckt und die Spule auf den Kern aufgebracht ist, wobei das Hinterende der Spule der Giebelwand zugewandt ist.

8. Vorrichtung nach Anspruch 7, wobei die zylindrische Wand (13) mit Ausschnitten (14) versehen ist.

9. Vorrichtung nach einem der Ansprüche 1, 3-8, wobei die Abschirmung (7) aus Ferrit besteht.

10. Vorrichtung nach einem der Ansprüche 1, 3-9, welche ferner einen Kunststoffkasten (5) umfasst, in dem die Spule (6) und die Abschirmung (7) derart angeordnet sind, dass sie in einem Abstand in der Größenordnung von Zentimetern von der Hand einer Bedienperson liegen, wenn die Bedienperson die Übertragungsvorrichtung (1; 19, 20) während der Bedienung hält.

11. Verwendung der Vorrichtung nach einem der Ansprüche 1-10 zur Übertragung des Wechselmagnetfeldes zu einem in einen Körper eines Menschen oder eines Tiers implantierten Empfänger, wobei der implantierte Empfänger (3; 15; 21) in der Lage ist, Energie aus dem Wechselmagnetfeld zu entnehmen, die zu einer Energie verbrauchenden medizinischen Vorrichtung geliefert werden soll, die in den Körper des Menschen oder des Tiers implantiert ist.

## Revendications

1. Appareil de transfert sans fil d'énergie provenant de l'extérieur d'un corps humain ou d'un corps d'animal dans un dispositif (17) médical consommant de l'énergie implanté dans le corps humain ou le corps d'un animal, comprenant un dispositif (1 ; 9, 20) de transmission pouvant être commandé de l'extérieur du corps humain ou du corps d'un animal pour la transmission d'un champ magnétique alternatif, un récepteur (3 ; 15 ; 21) implantable dans le corps humain ou le corps d'un animal destiné à recevoir et à tirer de l'énergie du champ magnétique alternatif à fournir au dispositif médical (17) consommant de l'énergie, le dispositif de transmission comprenant au moins une bobine (6) pour générer le champ magnétique alternatif dans une direction souhaitée vers le récepteur, la bobine ayant une extension longitudinale, une extrémité avant à diriger vers le récepteur et une extrémité arrière à diriger loin du récepteur, et au moins un écran (7 ; 12) adapté pour protéger l'environnement du champ magnétique alternatif généré par la bobine (6), l'écran comprenant un boîtier magnétisable (9) entourant l'extrémité arrière de la bobine, **caractérisé en ce que** l'écran comprend un noyau magnétisable (8) s'étendant dans la bobine (6), et **en ce que** le boîtier magnétisable (9) est intégré dans le noyau et entoure le pourtour de la bobine le long d'au moins une partie de l'extension longitudinale de la bobine.

2. Appareil selon la revendication 1, dans lequel le dispositif de transmission comprend un premier émetteur (19) et un deuxième émetteur (20), le premier et le deuxième émetteurs comprennent respectivement des première et deuxième bobines (6) destinées à générer des champs magnétiques alternatifs respectifs dans deux directions différentes vers le récepteur (21), chacune des première et deuxième bobines ayant une extension longitudinale, une extrémité avant à diriger vers le récepteur et une extrémité arrière à diriger loin du récepteur, et les premier et deuxième émetteurs comprennent des premier et deuxième écrans (9) adaptés pour protéger l'environnement des champs magnétiques alternatifs, chacun des premier et deuxième écrans comprenant un noyau magnétisable (8) s'étendant dans la bobine et un boîtier magnétisable (9) intégré dans le noyau et entourant l'extrémité arrière de la bobine et le pourtour de la bobine le long d'au moins une partie de l'extension longitudinale de la bobine.

3. Appareil selon la revendication 1, dans lequel le boîtier (9) entoure complètement la bobine (6) sauf l'extrémité avant de celle-ci.

4. Appareil selon la revendication 3, dans lequel le noyau (8) s'étend entièrement le long de l'extension longitudinale de la bobine (6).

5. Appareil selon la revendication 1, dans lequel le boîtier (9) entoure le pourtour de la bobine (6) le long d'une partie de l'extension longitudinale de la bobine.

6. Appareil selon la revendication 5, dans lequel le noyau (8) et/ou la bobine (6) s'étend au-delà du boîtier (9) le long de l'extension longitudinale de la bobine, telle que vue dans la direction vers l'extrémité avant de la bobine.

7. Appareil selon l'une quelconque des revendications 1, 3 à 6, dans lequel le boîtier (9) comprend une paroi cylindrique circulaire (10 ; 13) et une paroi pignon (11) circulaire jointe à la paroi cylindrique, le noyau (8) s'étend de manière centrale dans la paroi cylindrique à partir de la paroi pignon, et la bobine est appliquée sur le noyau avec l'extrémité arrière de la bobine faisant face à la paroi pignon.

8. Appareil selon la revendication 7, dans lequel la paroi cylindrique (13) est pourvue de découpes (14).

9. Appareil selon l'une quelconque des revendications 1, 3 à 8 ; dans lequel l'écran (7) est composé de ferrite.

10. Appareil selon l'une quelconque des revendications 1, 3 à 9, comprenant en outre un boîtier en plastique (5), dans laquelle la bobine (6) et l'écran (7) sont agencés de sorte qu'ils sont situés loin, de l'ordre de plusieurs centimètres, d'une main d'opérateur, quand l'opérateur tient le dispositif (1 ; 19, 20) de transmission pendant le fonctionnement.

11. Utilisation de l'appareil selon l'une quelconque des revendications 1 à 10, pour transmettre le champ magnétique alternatif à un récepteur implanté dans un corps humain ou un corps d'animal, le récepteur implanté (3 ; 15 ; 21) étant capable de tirer de l'énergie du champ magnétique alternatif pour alimenter un dispositif médical consommant de l'énergie dans le corps humain ou le corps animal.
